# EUROPEAN PATENT APPLICATION

(11) **EP 3 138 499 A1**
(43) Date of publication of application: **08.03.2017**
(21) Application number: 15785937.2
(22) Date of filing: 12.03.2015
(51) Int. Cl.: A61B 8/00, A61B 8/14

(54) **PORTABLE ULTRASONIC DIAGNOSTIC DEVICE HAVING LOW POWER MODE AND METHOD FOR PERFORMING SAME**

(30) Priority: 28.04.2014 KR 20140050662
(71) Applicant: Healcerion Co. Ltd., SEOUL 152-848 (KR)
(72) Inventor: RYU, Jeong Won, Seoul 137-140 (KR); CHOUNG, You Chan, Seoul 122-807 (KR); CHUNG, Wook Jin, Seoul 135-950 (KR)
(74) Representative: Groth & Co. KB
(86) International application number: PCT/KR2015/002401
(87) International publication number: WO 2015/167121

(57) **Abstract**

The present invention relates to a portable ultrasonic diagnostic device having a low power mode and a method for performing the same, and the portable ultrasonic diagnostic device having a low power mode, according to the present invention, comprises: an ultrasonic probe which transmits an ultrasonic signal to an object to be inspected, and then, which receives echo signals reflected from the object to be inspected; a beamformer which collects the echo signals reflected from the ultrasonic probe so as to generate frame data; a scan conversion unit which scans and converts the frame data generated in the beamformer so as to form ultrasonic images; a display unit provided with a display screen on which the scanned and converted ultrasonic images are displayed; a memory for storing image data on an image if an ultrasonic diagnosis is not carried on the image among the ultrasonic images of the display unit; an ultrasonic image comparison analyzer for comparing and analyzing the ultrasonic images of the display unit and the image data stored in the memory; and a low power mode control unit for enabling the whole circuit to enter a low power mode if it is determined that an ultrasonic image displayed on the display unit by the ultrasonic image comparison analyzer is an ultrasonic image for which an ultrasonic diagnosis has not been carried out. Thus, the present invention has the advantages of providing a portable ultrasonic diagnostic device having a low power mode and a method for performing the same, which can periodically analyze the brightness of a specific area of a display unit on which an ultrasonic image is displayed and can convert a mode into a low power mode if an ultrasonic diagnosis is not carried out, thereby reducing overall power consumption.

## Description

### [Technical Field]

The present invention relates to a portable ultrasonic diagnostic apparatus and a method of operating the same, and more particularly, a portable ultrasonic diagnostic apparatus having a low power mode and a method of operating the same, capable of reducing overall power consumption by periodically analyzing the brightness of a certain area of a display portion on which an ultrasonic image is displayed and converting a mode into a low power mode when ultrasonic diagnosis is not performed.

### [Background Art]

With noninvasive and nondestructive properties, an ultrasonic diagnostic apparatus is generally used in the medical field to obtain information of the inside of a subject. Since it is possible to provide a high-resolution image of internal organizations of the subject to a doctor without surgical operations of directly incising and observing the subject, an ultrasonic diagnostic system is very importantly used in the medical field.

An ultrasonic diagnostic apparatus is a system which transmits an ultrasonic signal from a body surface of a subject toward a target portion inside the subject, extracts information from a reflected ultrasonic signal, and obtains an image of a section of soft tissue or a blood flow in a noninvasive manner.

Compared with other imaging diagnostic apparatuses such as an X-ray inspection apparatus, a computerized tomography (CT) scanner, a magnetic resonance image (MRI) scanner, and a nuclear medicine inspection apparatus, since having a small size, being cheap, being capable of displaying in real time, and having excellent safety without being exposed to X-rays, an ultrasonic diagnostic system described above is generally used to diagnose hearts, internal organs in an abdominal cavity, urinary systems, and genital organs.

Since general ultrasonic diagnostic apparatuses use an alternating current (AC) power source which always supplies power, lacking of power does not occur. However, recently, as portable ultrasonic diagnostic apparatuses using a battery with limited power as a power source have been used, due to higher power consumption than general portable electronic devices, interest in a low power technology for maximally providing use time with minimal power has been increased.

### [Disclosure of Invention]

### [Technical Problem]

The present invention provides a portable ultrasonic diagnostic apparatus having a low power mode and a method for operating the same, capable of reducing overall power consumption by periodically analyzing the brightness of a certain area of a display portion on which an ultrasonic image is displayed and converting a mode into a low power mode when ultrasonic diagnosis is not performed.

### [Technical Solution]

One aspect of the present invention provides a portable ultrasonic diagnostic apparatus having a low power mode, including an ultrasonic probe which transmits ultrasonic signals to a subject and then receives echo signals reflected from the subject, a beamformer which collects the echo signals from the ultrasonic probe and generates frame data, a scan conversion portion which scans and converts the frame data generated by the beamformer and forms ultrasonic images, a display portion which includes a display screen on which the scanned and converted ultrasonic images are displayed, a memory which stores image data on an image when ultrasonic diagnosis is not performed on the image among the ultrasonic images of the display portion, an ultrasonic image comparative analyzer which compares and analyzes the ultrasonic images of the display portion and the image data stored in the memory, and a low power mode control portion which enables the whole circuit to enter a low power mode when it is determined by the ultrasonic image comparative analyzer that an ultrasonic image displayed on the display portion is an ultrasonic image of a case in which ultrasonic diagnosis is not performed.

The display screen of the display portion may include a main screen on which an ultrasonic image is displayed and an interface screen which receives a user command.

The main screen may include a first area which has a relatively higher level of illumination than other areas because light is concentrated when the ultrasonic diagnosis is not performed and a second area formed as a dark screen which has a similar level of illumination overall and is difficult to perform imagery interpretation when the ultrasonic probe does not receive echo signals.

The portable ultrasonic diagnostic apparatus may include a timer for periodically comparing and analyzing the ultrasonic image of the display portion with the image data stored in the memory and returning to a normal power mode from the low power mode after a certain time passes.

The low power mode may be a state in which power is not applied except a control circuit portion controlling overall operations.

Another aspect of the present invention provides a method of operating a portable ultrasonic diagnostic apparatus, including a first operation of collecting an ultrasonic image of a subject, a second operation of comparing the ultrasonic image of the subject with image data of a case in which the ultrasonic diagnosis is not performed, a third operation of returning to the first operation when the ultrasonic diagnosis is performed in the second operation and coming into a low power mode when the ultrasonic diagnosis is not performed, a fourth operation of standing by for a certain time when it comes into the low power mode in the third operation, and a fifth operation of releasing the low power mode after a certain time passes in the fourth operation and returning to the first operation.

The second operation of comparing the ultrasonic image of the subject with the image data of the case in which the ultrasonic diagnosis is not performed may be an operation of obtaining and comparing the ultrasonic image of the subject with differential images of the image data stored in the memory.

### [Advantageous Effects]

As described above, according to embodiments of the present invention, there are provided a portable ultrasonic diagnostic device having a low power mode and a method of operating the same, capable of reducing overall power consumption by periodically analyzing the brightness of a certain area of a display portion on which an ultrasonic image is displayed and converting a mode into a low power mode when ultrasonic diagnosis is not performed.

### [Brief Description of Drawings]

FIG. 1 is a block diagram of a portable ultrasonic diagnostic apparatus having a low power mode according to an embodiment of the present invention.
FIG. 2 is a flowchart illustrating a method of operating a portable ultrasonic diagnostic apparatus having a low power mode according to an embodiment of the present invention.
FIG. 3 illustrates a display screen which displays an ultrasonic image of a case of diagnosing a subject using ultrasound according to an embodiment of the present invention.
FIG. 4 illustrates a display screen of a case in which a subject is not diagnosed using ultrasound according to an embodiment of the present invention.

### [Mode for Invention]

Embodiments of the present invention are provided to more completely explain the present invention to one of ordinary skill in the art. The following embodiments may be modified into various other forms and the scope of the present invention is not limited thereto. The embodiments are provided to make the disclosure full and complete and to completely convey the concept to those skilled in the art.

The terms used herein are to explain particular embodiments but do not intend to limit the present invention. As used herein, singular expressions, unless contextually defined otherwise, may include plural expressions. Also, the terms "comprise" and/or "comprising" are used herein to specify the present of stated shapes, numbers, steps, operations, members, elements, and/or groups thereof but don not preclude the presence or addition of one or more other shapes, numbers, operations, members, elements and/or groups thereof. As used herein, the term "and/or" includes any and all combinations or one of a plurality of associated listed items.

It should be understood that although the terms "first", "second", etc. are used herein to describe various members, areas, layers, and/or portions, these members, areas, layers and/or portions are not limited by these terms. These terms do not mean particular order, top and bottom, or ratings but are used only to distinguish one member, area, or portion from another member, area, or portion. Accordingly, a first member, area, or portion which will be described below may be referred to as a second member, area, or portion without departing from the scope of the present invention.

Meanwhile, an ultrasonic diagnostic apparatus according to an embodiment of the present invention includes an ultrasonic probe, a beamformer, a scan conversion portion, and a display portion.

The ultrasonic probe transmits an ultrasonic signal to a subject and forms a reception signal by receiving an echo signal reflected from the subject. Also, the ultrasonic probe includes at least one transducer element which operates to transduce an ultrasonic signal and an electric signal into each other.

The beamformer analog/digital-converts and time-delays the reception signal provided from the ultrasonic probe considering a position and a focused point of each of the transducer elements and forms frame data by adding up time-delayed digital signals.

The scan converter performs scan conversion on frame data to be displayed on a display screen of the display portion.

The display portion displays ultrasonic data which is scan-converted as an ultrasonic image on the display screen.

Hereinafter, an exemplary embodiment of the present invention will be described in detail with reference to the attached drawings.

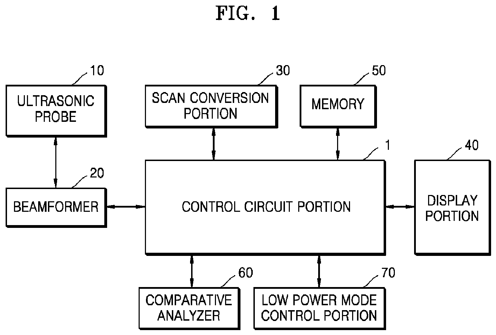

FIG. 1 is a block diagram of a portable ultrasonic diagnostic apparatus having a low power mode according to an embodiment of the present invention.

As shown in the drawings, the portable ultrasonic diagnostic apparatus having a low power mode according to the embodiment of the present invention may include an ultrasonic probe 10, a beamformer 20, a scan conversion portion 30, a display portion 40, a memory 50, an ultrasonic image comparative analyzer 60, and a low power mode control portion 70 and the whole circuit operation may be controlled by a control circuit portion 1.

In more detail, the ultrasonic probe 10 may transmit an ultrasonic signal to a subject and then may receive an echo signal reflected from the subject.

The beamformer 20 may collect the echo signal from the ultrasonic probe 10 and may generate frame data.

The scan conversion portion 30 may perform scan conversion on the frame data generated by the beamformer and may form an ultrasonic image.

The display portion 40 may include a display screen on which the scan-converted ultrasonic image is displayed.

Here, the display portion 40 may be provided at an external device such as a personal computer (PC), a smart phone, a tablet type device, a pad type device, personal digital assistants (PDA), etc. connected through wired or wireless communication.

The memory 50 may store image data with respect to an image of a case ultrasonic diagnosis is not performed, among the ultrasonic image of the display portion 40.

The ultrasonic image comparative analyzer 60 may compare and analyze the ultrasonic image of the display portion 40 with the image data stored in the memory 50.

The low power mode control portion 70 may allow the whole circuit to come into a low power mode when the ultrasonic image displayed on the display portion 40 is determined by the ultrasonic image comparative analyzer 60 as an ultrasonic image of a case ultrasonic diagnosis is not performed.

Meanwhile, the portable ultrasonic diagnostic apparatus having a low power mode according to the embodiment of the present invention may further include a timer (not shown).

By the timer, it is possible to periodically compare and analyze the ultrasonic image of the display portion 40 with the image data stored in the memory 50 and to return from a low power mode to a normal power mode after a certain time.

Here, in the embodiment of the present invention, the low power mode means a state in which power is not applied except a control circuit portion 1 but is not limited thereto and power may be controlled using various other methods such as cutting off only power for a display portion, etc.

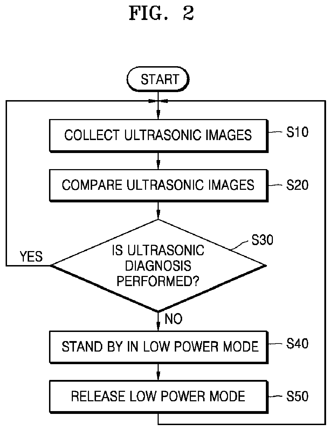

FIG. 2 is a flowchart illustrating a method of operating a portable ultrasonic diagnostic apparatus having a low power mode according to an embodiment of the present invention.

As shown in the drawings, the method of operating the portable ultrasonic diagnostic apparatus having a low power mode according to the embodiment of the present invention may include a first operation (S10) of collecting ultrasonic images, a second operation (S20) of comparing the ultrasonic images, a third operation (S30) of determining whether ultrasonic diagnosis is performed, a fourth operation (S40) of coming into a low power mode when ultrasonic diagnosis is not performed, and a fifth operation (S50) of releasing the low power mode after a certain time passes.

Referring to FIG. 1 described above, the flowchart shown in FIG. 2 will be described in detail as follows.

First, the first operation (S10) of collecting the ultrasonic image indicates an operation of collecting ultrasonic images of a subject using the beamformer 20.

Next, the second operation (S20) of comparing the ultrasonic images indicates an operation of comparing, by the ultrasonic image comparative analyzer 60, the ultrasonic images of the subject collected by the beamformer 20 with image data of a case in which ultrasonic diagnosis is not performed, which is stored in the memory 50.

Next, the third operation (S30) of determining whether the ultrasonic diagnosis is performed indicates an operation of returning to the first operation (S10) of collecting the ultrasonic images of the subject by the beamformer 20 when a result of comparison of the second operation (S20) performed by the ultrasonic image comparative analyzer 60 and coming into the low power mode, by the low power mode control portion 70 when it is determined that the ultrasonic diagnosis is not performed.

Next, the fourth operation (S40) of coming into the low power mode when the ultrasonic diagnosis is not performed may be embodied as a standby operation for a certain time preset in a timer (not shown) after coming into the low power mode by the low power mode control portion 70 in the third operation (S30). Also, a user may change setting of the time to change a standby time.

Next, the fifth operation (S50) of releasing the low power mode after the certain time passes indicates an operation of releasing the low power mode when the certain time preset in the time after coming into the low power mode in the fourth operation (S40) and returning to the first operation (S10) of collecting the ultrasonic images by the beamformer 20.

Meanwhile, the second operation (S20) of comparing the ultrasonic images of the subject with the image data of the case in which the ultrasonic diagnosis is not performed is an operation of obtaining and comparing the ultrasonic images of the subject formed by the scan conversion portion 30 with differential images of the image data stored in the memory 50.

That is, whether the ultrasonic diagnosis is performed is determined using an amount of data included in differential images. Here, it is determined that the larger amount of data forming the differential images, the larger difference between the two compared images.

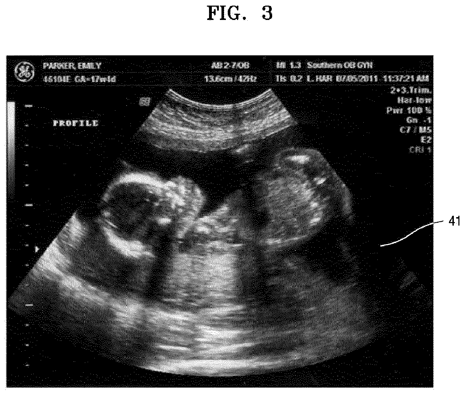

FIG. 3 illustrates a display screen which displays an ultrasonic image of a case of diagnosing a subject using ultrasound according to an embodiment of the present invention.

A screen shown in the drawing indicates an ultrasonic image displayed on a display screen when a pregnant woman is diagnosed.

As shown in the drawing, the display screen on which the ultrasonic image is displayed includes a main screen 41 on which the ultrasonic image is displayed and an interface screen (not shown) for receiving a user command.

It is possible to observe diagnosis information for the subject seeing the displayed ultrasonic image through the main screen 41 and to select or change the menu of the ultrasonic image through the interface screen.

As described above, it may be known that when the subject is diagnosed using ultrasound, bright and dark areas are evenly distributed over the ultrasonic image displayed on the display screen and image patterns in various shapes are formed depending on a diagnosed part.

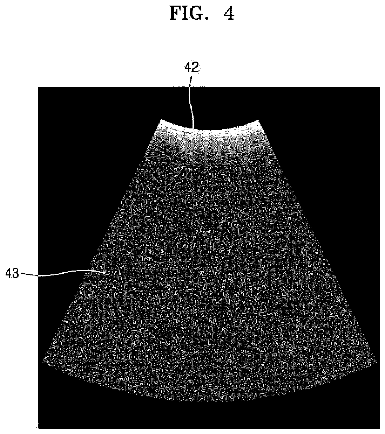

FIG. 4 illustrates a display screen of a case in which a subject is not diagnosed using ultrasound according to an embodiment of the present invention.

As shown in the drawing, the main screen 41 on which the ultrasonic image is displayed includes a first area 42 which shines brightly at the uppermost end portion of the display screen and a second area 43 which is dark overall with similar brightness.

Here, in the first area 42, since a front portion is exposed in the air when the ultrasonic probe 10 of FIG. 1 does not perform ultrasonic diagnosis, light is concentrated due to total reflection which occurs at the ultrasonic probe 10 and a level of illumination may be formed relatively higher than other areas.

Also, since the ultrasonic probe 10 of FIG. 1 does not receive an echo signal when the ultrasonic probe 10 does not perform ultrasonic diagnosis, the second area 43 may be formed as a dark screen which has a similar level of illumination overall and is difficult to perform imagery interpretation.

Hereinafter, an operating method according to another embodiment of the present invention will be described.

Whether a shade difference between preset areas for a time not in contact with a human body and standing by exceeds a threshold is determined, and when there is a difference of a certain time or more between the shade difference between the both areas and the threshold, it is determined as a human non-contact state and comes into a low power mode. Determining of the areas compared in the shade difference is changeable according to a probe type.

In FIG. 4, whether a human body is not touched may be determined using whether the shade difference between the both areas exceeds the preset threshold by comparing a part or all of the first area 42 and a part or all of the second area 43.

When the human body is touched after coming into the low power mode, the low power mode is released and it operates in a normal mode.

As described above, the present invention has an effect of providing a portable ultrasonic diagnostic device having a low power mode and a method for operating the same, capable of reducing overall power consumption by periodically analyzing the brightness of a certain area of a display portion on which an ultrasonic image is displayed and converting a mode into a low power mode when ultrasonic diagnosis is not performed.

While the present invention has been described in detail, it should be known that the embodiments described above are only exemplary and not limitative and it will be understood that various changes in form and details may be made therein without departing from the spirit and scope of the present invention as defined by the following claims.

## Claims

1. A portable ultrasonic diagnostic apparatus having a low power mode, comprising:
an ultrasonic probe which transmits ultrasonic signals to a subject and then receives echo signals reflected from the subject;
a beamformer which collects the echo signals from the ultrasonic probe and generates frame data;
a scan conversion portion which scans and converts the frame data generated by the beamformer and forms ultrasonic images;
a display portion which comprises a display screen on which the scanned and converted ultrasonic images are displayed;
a memory which stores image data on an image when ultrasonic diagnosis is not performed on the image among the ultrasonic images of the display portion;
an ultrasonic image comparative analyzer which compares and analyzes the ultrasonic images of the display portion and the image data stored in the memory; and
a low power mode control portion which enables the whole circuit to enter a low power mode when it is determined by the ultrasonic image comparative analyzer that an ultrasonic image displayed on the display portion is an ultrasonic image of a case in which ultrasonic diagnosis is not performed.

2. The portable ultrasonic diagnostic apparatus of claim 1, wherein the display screen of the display portion comprises a main screen on which an ultrasonic image is displayed and an interface screen which receives a user command, and wherein the main screen comprises:
a first area which has a relatively higher level of illumination than other areas because light is concentrated when the ultrasonic diagnosis is not performed; and
a second area formed as a dark screen which has a similar level of illumination overall and is difficult to perform imagery interpretation when the ultrasonic probe does not receive echo signals.

3. The portable ultrasonic diagnostic apparatus of claim 1, comprising a timer for periodically comparing and analyzing the ultrasonic image of the display portion with the image data stored in the memory and returning to a normal power mode from the low power mode after a certain time passes.

4. The portable ultrasonic diagnostic apparatus of claim 1, wherein the low power mode is a state in which power is not applied except a control circuit portion controlling overall operations.

5. A method of operating a portable ultrasonic diagnostic apparatus, comprising:
a first operation of collecting an ultrasonic image of a subject;
a second operation of comparing the ultrasonic image of the subject with image data of a case in which the ultrasonic diagnosis is not performed;
a third operation of returning to the first operation when the ultrasonic diagnosis is performed in the second operation and coming into a low power mode when the ultrasonic diagnosis is not performed;
a fourth operation of standing by for a certain time when it comes into the low power mode in the third operation; and
a fifth operation of releasing the low power mode after a certain time passes in the fourth operation and returning to the first operation.

6. The method of claim 5, wherein the second operation of comparing the ultrasonic image of the subject with the image data of the case in which the ultrasonic diagnosis is not performed is an operation of obtaining and comparing the ultrasonic image of the subject with differential images of the image data stored in the memory.
